# EUROPEAN PATENT SPECIFICATION

(11) **EP 4 011 436 B1**
(45) Date of publication and mention of the grant of the patent: **12.07.2023**
(21) Application number: 20212860.9
(22) Date of filing: 09.12.2020
(51) Int. Cl.: A61N 1/04, A61N 1/32, A61N 1/36, C12N 13/00, C12M 1/42

(54) **PULSED ELECTRIC FIELD TREATMENT OF BIOLOGICAL CELLS**
BEHANDLUNG BIOLOGISCHER ZELLEN MIT EINEM GEPULSTEN ELEKTRISCHEN FELD
TRAITEMENT DE CELLULES BIOLOGIQUES PAR CHAMP ÉLECTRIQUE PULSÉ

(43) Date of publication of application: 15.06.2022
(73) Proprietor: Bühler AG, 9240 Uzwil (CH)
(72) Inventor: BUCHMANN, Leandro, 8408 Winterthur (CH)
(74) Representative: Leimgruber, Fabian Alfred Rupert

(56) References cited:
- EP-A1- 2 147 697
- EP-A2- 3 290 082
- WO-A2-2020/146702
- RAM ANAND VADLAMANI ET AL: "Nanosecond pulsed electric field induced proliferation and differentiation of osteoblasts and myoblasts", JOURNAL OF THE ROYAL SOCIETY. INTERFACE, vol. 16, no. 155, 1 June 2019 (2019-06-01) , page 20190079, XP055723796, GB ISSN: 1742-5689, DOI: 10.1098/rsif.2019.0079

## Description

### FIELD OF THE INVENTION

The present invention as defined in claims 1, 3 and 7 relates to methods of increasing metabolic activity and stimulating cell proliferation of biological cells through the use of pulsed electric field (PEF) treatment, especially through the use of nanosecond pulsed electric field (nsPEF) treatment.

### BACKGROUND OF THE INVENTION

Pulsed electric field (PEF) processing is a growing field in the area of electromagnetic technologies for medical, environmental, and food applications (see Perspective article by Buchmann & Mathys, Frontiers in Bioengineering and Biotechnology, vol. 7, article 265, 16 October 2019). PEF treatment is based on the formation of a potential difference across a conductive biological material between two electrodes, creating an electric field that depends on the applied electric voltage, the shape of the electrodes, and the gap between electrodes.

PEF processing can be divided into conventional PEF processing in the range of micro- to milliseconds and nanosecond (nsPEF) processing, in which high electric fields (10-100 kV cm⁻¹) are applied for 1-300 ns. nsPEF induces intracellular effects, distinct from the pronounced effects of conventional PEF on the cell membrane. Thereby, innovative applications and novel process windows are possible, while similar components for both treatments in batch and continuous mode are required. In both cases, the resulting electropermeabilization increases the mass transfer of molecules and ions. Depending on process parameters, a reversible or irreversible effect can be induced. Most current applications are focused on irreversible electropermeabilization, including non (minimal)-thermal pasteurization, enhanced drying rates, increased extraction yields, tissue softening as well as electrochemotherapy, and tumor ablation. Reversible electropermeabilization is typically used in molecular biology for the introduction of specific molecules, such as plasmids and antibodies, *in vivo.* However, the mechanisms underlying the PEF/ nsPEF induced effects are still the subject of intensive research.

### TECHNICAL PROBLEMS UNDERLYING THE PRESENT INVENTION

The present inventors have devised improved processes of pulsed electric field (PEF) treatment and nanosecond pulsed electric field (nsPEF) treatment of prokaryotic and eukaryotic cells.

The processes of the present invention provide an increased productivity of the treated cells due to a more homogenous cellular response; allow for both an intracellular and an extracellular manipulation of cells; result in an increased biomass yield and an increased production of specific compounds due to a more targeted and more ubiquitous application of the electric field; allows specific manipulation of cells in a wide range of sizes; and can be easily adapted to an industrial scale.

This summary does not necessarily describe all advantages achieved and all problems solved by the present invention. EP 2 147 697; WO 2020/146702; EP 3 290 082 and RAM ANAND VADLAMANI ET AL: "Nanosecond pulsed electric field induced proliferation and differentiation of osteoblasts and myoblasts",JOURNAL OF THE ROYAL SOCIETY. INTERFACE, vol. 16, no. 155, 1 June 2019 (2019-06-01) disclose known PEF systems for treating cells.

### SUMMARY OF THE INVENTION

The present invention is set out in the appended claims. The embodiments, examples or aspects according to the present description which do not fall within the scope of said claims are provided for illustrative purposes only and do not form part of the present invention. In a first aspect the present description relates to a method of increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby increasing metabolic activity of the biological cells;
   wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and
   wherein the pulses of electricity, when reaching the target voltage, have an electric field strength of 0.5 kV/cm to 50 kV/cm, preferably of 1.0 kV/cm to 30 kV/cm.

In a second aspect the present description relates to a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 2 to 30 pulses, preferably from 2 to 12 pulses, of electricity between the electrodes, thereby stimulating cell proliferation;
   wherein a voltage increase between the two electrodes from 10 % to 90 % of a target voltage of the pulses of electricity takes place within a time period of 1 ns to 25 ns, preferably within a time period of 3 ns to 15 ns; wherein the pulses of electricity have a pulse duration of between 50 ns to 300 ns, preferably of about 100 ns; and wherein the pulses of electricity, when reaching the target voltage, have an electric field strength of 1.0 kV/cm to 30 kV/cm, preferably of 5.0 kV/cm to 20 kV/cm.

In a third aspect the present description relates to a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby stimulating cell proliferation;
   wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and
   wherein the pulses of electricity, when reaching the target voltage, have an electric field strength of 0.5 kV/cm to 50 kV/cm, preferably of 1.0 kV/cm to 30 kV/cm;
wherein the biological cells are selected from the group consisting of bacterial cells, yeast cells, fungal cells, microalgae cells, plant cells, animal cells, and human cells.

In a fourth aspect the present description relates to a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby stimulating cell proliferation;
   wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and
   wherein the pulses of electricity, when reaching the target voltage, have an electric field strength of 0.5 kV/cm to 50 kV/cm, preferably of 1.0 kV/cm to 30 kV/cm;
   wherein step (c) is carried out in a recirculation process.

In a fifth aspect the present description relates to a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby stimulating cell proliferation;
   wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and
   wherein the pulses of electricity, when reaching the target voltage, have an electric field strength of 0.5 kV/cm to 50 kV/cm, preferably of 1.0 kV/cm to 30 kV/cm;
   wherein step (c) is carried out in a batch process.

### DETAILED DESCRIPTION OF THE INVENTION

### Definitions

The present invention is set out in claims 1, 3 and 7.

As used herein, the term "about" refers to numerical values ranging from 5% below the indicated numerical value to 5% above the indicated numerical value. For example, a pulse duration of *"about 100 ns"* encompasses a pulse duration ranging from 95 ns to about 105 ns.

### Aspects of the Invention

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect the present description provides a method of increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses of electricity between the electrodes, thereby increasing metabolic activity of the biological cells;
   wherein a voltage increase between the two electrodes from 10 % to 90 % of a target voltage of the pulses of electricity takes place within a time period of 0.1 to 100 ns,
   wherein the pulses of electricity have a pulse duration of between 5 to 5000 ns, and
   wherein the pulses of electricity, when reaching the target voltage, have an electric field strength of 0.5 kV/cm to 50 kV/cm.

The present disclosure demonstrates that the described processes of PEF treatment are not only suitable to stimulate proliferation of biological cells but are also suitable to increase metabolic activity of biological cells and achieve higher yields of specific endogenous or exogenous compounds.

In a second aspect the present description provides a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 2 to 30 pulses of electricity between the electrodes, thereby stimulating cell proliferation;
   wherein a voltage increase between the two electrodes from 10 % to 90 % of a target voltage of the pulses of electricity takes place within a time period of 1 ns to 25 ns,
   wherein the pulses of electricity have a pulse duration of between 50 ns to 300 ns, and
   wherein the pulses of electricity, when reaching the target voltage, have an electric field strength of 1.0 kV/cm to 30 kV/cm.

Without wishing to be bound by a particularly theory, it is the inventors' opinion that the physical parameters used in the second aspect will lead to an increased productivity due to a more homogenous cellular response; and/or will allow both an intracellular and an extracellular manipulation of cells; and/or will result in an increased biomass yield and/or an increased production of specific compounds due to a more targeted and more ubiquitous application of the electric field; and/or will allow specific manipulation of cells in the range from 1 µm to 70 mm in size (preferably from 1 µm to 10 mm); and/or will allow the adaptation of the process to an industrial scale.

In a third aspect the present description provides a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses of electricity between the electrodes, thereby stimulating cell proliferation;
   wherein a voltage increase between the two electrodes from 10 % to 90 % of a target voltage of the pulses of electricity takes place within a time period of 0.1 to 100 ns,
   wherein the pulses of electricity have a pulse duration of between 5 to 5000 ns, and
   wherein the pulses of electricity, when reaching the target voltage, have an electric field strength of 0.5 kV/cm to 50 kV/cm;
   wherein the biological cells are selected from the group consisting of bacterial cells, yeast cells, fungal cells, microalgae cells, plant cells, animal cells, and human cells.

In a fourth aspect the present description provides a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses of electricity between the electrodes, thereby stimulating cell proliferation;
   wherein a voltage increase between the two electrodes from 10 % to 90 % of a target voltage of the pulses of electricity takes place within a time period of 0.1 to 100 ns,
   wherein the pulses of electricity have a pulse duration of between 5 to 5000 ns, and
   wherein the pulses of electricity, when reaching the target voltage, have an electric field strength of 0.5 kV/cm to 50 kV/cm;
   wherein step (c) is carried out in a recirculation process.

Without wishing to be bound by a particularly theory, it is the inventors' opinion that a recirculation process facilitates the industrial implementation of the process of pulsed electric filed treatment.

In a fifth aspect the present description provides a method of stimulating cell proliferation and/or increasing metabolic activity of biological cells, said method comprising the following steps:
(a) providing biological cells and suspending the cells in an electrically conductive liquid, thereby forming a suspension,
(b) positioning the suspension between two electrodes, and
(c) applying from 1 to 100 pulses of electricity between the electrodes, thereby stimulating cell proliferation;
   wherein a voltage increase between the two electrodes from 10 % to 90 % of a target voltage of the pulses of electricity takes place within a time period of 0.1 to 100 ns,
   wherein the pulses of electricity have a pulse duration of between 5 to 5000 ns, and
   wherein the pulses of electricity, when reaching the target voltage, have an electric field strength of 0.5 kV/cm to 50 kV/cm;
   wherein step (c) is carried out in a batch process.

Without wishing to be bound by a particularly theory, it is the inventors' opinion that a batch process will reduce the overall mechanical and/or physical stress occurring during the electric field treatment of the cells.

In a preferred example of the first, third, fourth, or fifth aspect of the description, from 2 to 30 pulses of electricity are applied in step (c).

In a preferred example of any aspect of the invention, from 2 to 25 pulses, preferably from 2 to 20 pulses, more preferably from 2 to 15 pulses, and even more preferably from 2 to 12 pulses, for example 2, 3, 4, 5, 6, 7, 8, 9, 10, 11, or 12 pulses, are applied in step (c).

In a preferred example of the first, third, fourth, or fifth aspect of the description, the voltage increase between the two electrodes from 10 % to 90 % of a target voltage of the pulses of electricity takes place within a time period of 1 ns to 25 ns,

In a preferred example of any aspect of the description, the voltage increase between the two electrodes from 10 % to 90 % of a target voltage of the pulses of electricity takes place within a time period of 2 ns to 10 ns, more preferably within a time period of 3 ns to 15 ns.

In a preferred example of the first, third, fourth, or fifth aspect of the description, the pulses of electricity have a pulse duration of between 50 ns and 300 ns.

In a preferred example of any aspect of the description, the pulses of electricity have a pulse duration of between 60 ns and 250 ns, preferably of between 70 ns and 200 ns, more preferably of between 80 ns and 150 ns, more preferably of between 90 ns and 125 ns, and most preferably of about 100 ns.

In a preferred example of the first, third, fourth, or fifth aspect of the invention, the pulses of electricity, when reaching the target voltage, have an electric field strength of 1.0 kV/cm to 30 kV/cm.

In a preferred example of any aspect of the description, the pulses of electricity, when reaching the target voltage, have an electric field strength of 2 kV/cm to 28 kV/cm, preferably 3 kV/cm to 25 kV/cm, more preferably 4 kV to 23 kV/cm, and most preferably of 5 kV/cm to 20 kV/cm.

In a preferred example the first, third, fourth, or fifth aspect of the description, the potential difference between the electrodes is between 2 to 300 kV, preferably between 2 and 250 kV, more preferably between 2 and 200 kV, more preferably between 2 and 150 kV, more preferably, between 2 and 100 kV, more preferably between 2 and 50 kV, even more preferably between 2 and 40 kV, even more preferably between 3 and 30 kV, even more preferably between 4 and 20 kV, and most preferably between 5 and 10 kV.

In a preferred example the first, second, fourth, or fifth aspect of the description, the biological cells are selected from the group consisting of bacterial cells, yeast cells, fungal cells, microalgae cells, plant cells, animal cells, and human cells.

In a preferred example of any aspect of the description,
- the bacterial cells are selected from the group consisting of *Escherichia coli* and *Corynebacterium glutamicum*;
- the yeast cells are selected from the group consisting of *Pichiapastoris* and *Saccharomyces cerevisiae;*
- the microalgae cells are selected from the group consisting of *Galdieria sulphuraria* and *Aurantiochytrium limacinum;*
- the plant cells are selected from the group consisting of *Hordeum vulgare* and *Oryza sativa;* and
- the animal cells are selected from the group consisting of *Spodoptera frugiperda* and *Trichiplusia ni.*

In a preferred example of the first, second, or third aspect of the description, step (c) is carried out in a recirculation process.

In a preferred example of the first, second, or third aspect of the description, step (c) is carried out in a batch process.

The following figures and examples are merely illustrative of the present invention and should not be construed to limit the scope of the invention as defined by the appended claims in any way.

### BRIEF DESCRIPTION OF THE FIGURES

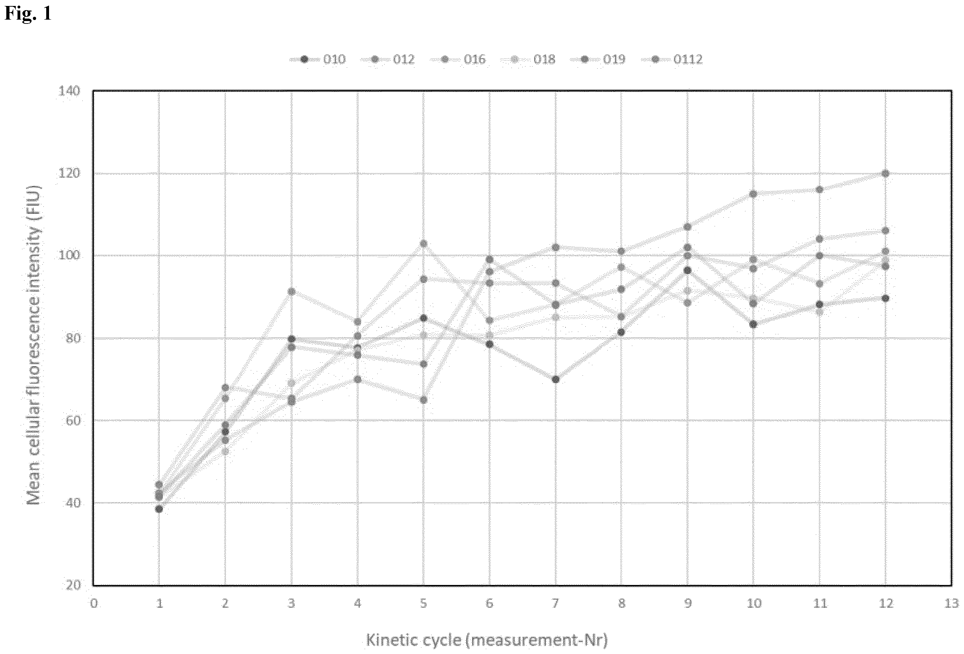

**Fig. 1****.: Metabolic activity of different *E. coli* samples determined by FDA (fluorescein diacetate) cleavage.** The *E. coli* cell samples received different numbers of electric pulses of a nanosecond pulsed electric field (nsPEF) treatment in a recirculation process. All *E. coli* cell samples exhibited an increase in metabolic activity due to the nsPEF treatment.

### EXAMPLES

### Example 1:

A twin bioreactor system was used for parallel cultivations. The parallel cultivations were inoculated with the same amount of pre-culture from the same feed train of the selected bacterial cell *Escherichia coli.* The reactor cultivations as well as the pre-culture/ inoculum were performed in the modified, defined MD-FB medium:
14.3 g L⁻¹ (pre-culture) and 28.7 g L⁻¹ glycerol 85%,
K₂HPO₄ 2.70 g L⁻¹,
KH₂PO₄ 13.2 g L⁻¹,
NaCl 2.04 g L⁻¹,
(NH₄)SO₄ 4.1 g L⁻¹,
Antifoam 205 0.05 g L⁻¹,
ddH₂O 920.8 g L⁻¹,
0.8 mL MgSO₄ * 7 H₂O stock (300 g L⁻¹),
10.0 mL Fe(III) citrate stock (10.0 g L⁻¹),
10 mL Na-EDTA * 2 H₂O stock (0.84 g L⁻¹),
2.8 mL Thiamine HCl stock (45.0 g L⁻¹),
2.9 mL TE stock (CoCl₂ ^{∗} 6 H₂O 0.16 g L⁻¹,
MnCl₂ ^{∗} 4 H₂O 1.42 g L⁻¹,
H₃BO₃ 0.01 g L⁻¹,
Na₂MoO₄ ^{∗} 2 H₂O 0.02 g L⁻¹,
CaCl₂ ^{∗} 2 H₂O 1.44 g L⁻¹,
AlCl₃ ^{∗} 6 H₂O 0.04 g L⁻¹,
ZnSO₄ ^{∗} 7 H₂O 0.87 g L⁻¹,
CuSO₄ ^{∗} 5 H₂O 1.55 g L⁻¹,
NiCl₂ ^{∗} 6 H₂O 0.01 g L⁻¹

Cultivation conditions are summarized in Table 1:

**Table 1:**

| **Parameter** | **Value** | **Unit** |
|---|---|---|
| Reactor working volume | 10 | [L] |
| pH | 7 | [-] |
| Aeration (sterile air) | 3 | [vvm] |
| Temperature | 30.0 | [°C] |
| Pressure | 0.5 | [bar] |
| Stirrer | 1200 | [rpm] |

Upon treatment the metabolic activity was expressed as the rate and total amount of individual cellular FDA (fluorescein diacetate) cleavage. Standard staining protocols were used in the experiments and analysed in a flow cytometer (Ehgartner D, Herwig C, Neutsch L. At-line determination of spore inoculum quality in Penicillium chrysogenum bioprocesses. Appl Microbiol Biotechnol. 2016;100(12):5363-73; Söderström BE. Vital staining of fungi in pure cultures and in soil with fluorescein diacetate. Soil Biol Biochem. 1977; 9:59-63; and Pekarsky, A., Veiter, L., Rajamanickam, V. et al. Production of a recombinant peroxidase in different glyco-engineered Pichia pastoris strains: a morphological and physiological comparison. Microb Cell Fact 17, 183 (2018)).

Table 2 summarizes the nsPEF conditions for the control and five different samples.

**Table 2:**

| **Sample No.** | 010 | 012 | 016 | 018 | 019 | 0112 |
|---|---|---|---|---|---|---|
| **Potential difference [kV]** | Control | 10 | 10 | 10 | 10 | 10 |
| **Pulse length [ns]** | | 100 | 100 | 100 | 100 | 100 |
| **Pulses of electricity per treatment passage [-]** | | 4 | 6 | 8 | 10 | 12 |
| **Electrode distance [cm]** | 0.5 | 0.5 | 0.5 | 0.5 | 0.5 | 0.5 |

An increase in metabolic activity was detected for all treated samples (see **Fig. 1**). Surprisingly, the greatest increase of metabolic activity was observed for sample no. 12, which received the lowest number of pulses (i.e. 4 pulses). Sample No. 012 exhibited an increase of metabolic activity as compared to control sample 010 from 90 FIU to 120 FIU, which corresponds to an increase of about 30%

### Example 2:

A twin bioreactor system was used for parallel cultivations. The parallel cultivations were inoculated with the same amount of pre-culture from the same feed train of selected yeast cell *Pichia pastoris.* The reactor cultivations were performed in the modified, defined medium (Hellwig et al., 2001):
35.29 g L⁻¹ Glycerol 85%,
K₂SO₄ 2.86 g L⁻¹,
KOH 0.64 g L⁻¹,
MgSO₄ ^{∗} 7 H₂O 2.32 g L⁻¹,
CaSO₄ ^{∗} 2 H₂O 0.17 g L⁻¹,
Na-EDTA ^{∗} 2 H₂O 0.6 g L⁻¹,
NaCl 0.22 g L⁻¹,
Antifoam 205 0.1 g L⁻¹,
H₃PO₄ (85% 7.19 g L⁻¹,
Water 860 g L⁻¹,
PTM1 stock 4.35 mL L⁻¹ (CuSO₄ 6.0 g L⁻¹, NaI 0.08 g L⁻¹, MnSO₄ ^{∗} H₂O 3.0 g L⁻¹, Na₂MoO₄ ^{∗} 2 H₂O 0.2 g L⁻¹, CoCl₂ ^{∗} 6 H₂O 0.5 g L⁻¹, ZnCl₂ 20.0 g L⁻¹, FeSO₄ ^{∗} 7 H₂O 65.0 g L⁻¹, H₂SO₄ (98%) 50 mL l⁻¹, H₃BO₃ 0.02 g L⁻¹),
Biotin stock 500 × 4.35 mL L⁻¹ (0.2 g L⁻¹)

Cultivation conditions are summarized in Table 3:

**Table 3:**

| **Parameter** | **Value** | **Unit** |
|---|---|---|
| Reactor working volume | 10 | [L] |
| pH | 7 | [-] |
| Aeration (sterile air) | 3 | [vvm] |
| Temperature | 30.0 | [°C] |
| Pressure | 0.5 | [bar] |
| Stirrer | 800 | [rpm] |

Upon treatment the metabolic activity was increased on an individual cell basis, based on the analysed parameters.

Table 4 summarizes the nsPEF conditions for the control and four different samples.

**Table 4:**

| **Sample No.** | 0 | 1 | 2 | 3 | 4 |
|---|---|---|---|---|---|
| **Potential difference [kV]** | Control | 5 | 5 | 5 | 5 |
| **Pulse length [ns]** | | 100 | 100 | 100 | 100 |
| **Pulses of electricity per treatment passage [-]** | | 2 | 3 | 4 | 6 |
| **Electrode distance [cm]** | 0.5 | 0.5 | 0.5 | 0.5 | 0.5 |

## Claims

1. An *in vitro* method of increasing metabolic activity of biological cells, said method comprising the following steps:
(a) suspending biological cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby increasing metabolic activity of the biological cells;
wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and wherein the pulses of electricity, when reaching the target voltage, have an electric field strength of 0.5 kV/cm to 50 kV/cm, preferably of 1.0 kV/cm to 30 kV/cm.

2. The method of claim 1, wherein the biological cells are selected from the group consisting of bacterial cells, yeast cells, fungal cells, microalgae cells, plant cells, animal cells, and human cells.

3. An *in vitro* method of stimulating cell proliferation and/or increasing metabolic activity of biological cells, said method comprising the following steps:
(a) suspending biological cells in an electrically conductive liquid, thereby forming a suspension,
(b) positioning the suspension between two electrodes, and
(c) applying from 1 to 100 pulses, preferably from 2 to 30 pulses, of electricity between the electrodes, thereby stimulating cell proliferation;
wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and wherein the pulses of electricity, when reaching the target voltage, have an electric field strength of 0.5 kV/cm to 50 kV/cm, preferably of 1.0 kV/cm to 30 kV/cm;
wherein the biological cells are selected from the group consisting of bacterial cells, yeast cells, fungal cells, microalgae cells, plant cells, and animal cells, wherein the animal cells are selected from the group consisting of *Spodoptera frugiperda* and *Trichoplusia ni.*

4. The method of claim 2 or 3, wherein
- the bacterial cells are selected from the group consisting of *Escherichia coli* and *Corynebacterium glutamicum;*
- the yeast cells are selected from the group consisting of *Pichiapastoris* and *Saccharomyces cerevisiae;*
- the microalgae cells are selected from the group consisting of *Galdieria sulphuraria* and *Aurantiochytrium limacinum;*
- the plant cells are selected from the group consisting of *Hordeum vulgare* and *Oryza sativa;* and
- the animal cells are selected from the group consisting of *Spodoptera frugiperda* and *Trichoplusia ni.*

5. The method of any one of claims 1 to 4, wherein step (c) is carried out in a recirculation process adapted to an industrial scale.

6. The method of any one of claims 1 to 4, wherein step (c) is carried out in a batch process.

7. An *in vitro* method of stimulating cell proliferation and/or increasing metabolic activity of biological cells, said method comprising the following steps:
(a) suspending biological cells s in an electrically conductive liquid, thereby forming a suspension,
(b) positioning the suspension between two electrodes, and
(c) applying from 1 to 100 pulses, preferably from 2 to 30 pulses, of electricity between the electrodes, thereby stimulating cell proliferation;
wherein a voltage increase between the two electrodes from 10 % to 90 % of a target voltage of the pulses of electricity takes place within a time period of 0.1 to 100 ns, preferably within a time period of 1 ns to 25 ns; wherein the pulses of electricity have a pulse duration of between 5 to 5000 ns, preferably of between 50 ns to 300 ns; and wherein the pulses of electricity, when reaching the target voltage, have an electric field strength of 0.5 kV/cm to 50 kV/cm, preferably of 1.0 kV/cm to 30 kV/cm;
wherein step (c) is carried out in a recirculation process adapted to an industrial scale.

8. The method of claim 7, wherein the biological cells are selected from the group consisting of bacterial cells, yeast cells, fungal cells, microalgae cells, plant cells, animal cells, and human cells.

9. The method of claim 8, wherein
- the bacterial cells are selected from the group consisting of *Escherichia coli* and *Corynebacterium glutamicum;*
- the yeast cells are selected from the group consisting of *Pichiapastoris* and *Saccharomyces cerevisiae;*
- the microalgae cells are selected from the group consisting of *Galdieria sulphuraria* and *Aurantiochytrium limacinum;*
- the plant cells are selected from the group consisting of *Hordeum vulgare* and *Oryza sativa;* and
- the animal cells are selected from the group consisting of *Spodoptera frugiperda* and *Trichoplusia ni.*

## Patentansprüche

1. *In*-*vitro*-Verfahren zur Erhöhung der Stoffwechselaktivität von biologischen Zellen, wobei das Verfahren die folgenden Schritte umfasst:
(a) Suspendieren von biologischen Zellen in einer elektrisch leitenden Flüssigkeit, um dadurch eine Suspension zu bilden,
(b) Positionieren der Suspension zwischen zwei Elektroden und
(c) Anlegen von 1 bis 100 elektrischen Impulsen, vorzugsweise 2 bis 30 elektrischen Impulsen zwischen den Elektroden, um dadurch die Stoffwechselaktivität der biologischen Zellen zu erhöhen;
wobei eine Spannungserhöhung zwischen den beiden Elektroden von 10 % bis 90 % einer Zielspannung der elektrischen Impulse innerhalb einer Zeitspanne von 0,1 bis 100 ns, vorzugsweise innerhalb einer Zeitspanne von 1 ns bis 25 ns stattfindet; wobei die elektrischen Impulse eine Impulsdauer von 5 bis 5000 ns, vorzugsweise 50 ns bis 300 ns aufweisen; und wobei die elektrischen Impulse bei Erreichen der Zielspannung eine elektrische Feldstärke von 0,5 kV/cm bis 50 kV/cm, vorzugsweise 1,0 kV/cm bis 30 kV/cm aufweisen.

2. Verfahren nach Anspruch 1, wobei die biologischen Zellen aus der Gruppe bestehend aus Bakterienzellen, Hefezellen, Pilzzellen, Mikroalgenzellen, Pflanzenzellen, Tierzellen und Menschenzellen ausgewählt werden.

3. *In*-*vitro*-Verfahren zur Stimulation der Zellproliferation und/oder Erhöhung der Stoffwechselaktivität von biologischen Zellen, wobei das Verfahren die folgenden Schritte umfasst:
(a) Suspendieren von biologischen Zellen in einer elektrisch leitenden Flüssigkeit, um dadurch eine Suspension zu bilden,
(b) Positionieren der Suspension zwischen zwei Elektroden und
(c) Anlegen von 1 bis 100 elektrischen Impulsen, vorzugsweise 2 bis 30 elektrischen Impulsen zwischen den Elektroden, um dadurch die Zellproliferation zu stimulieren;
wobei eine Spannungserhöhung zwischen den beiden Elektroden von 10 % bis 90 % einer Zielspannung der elektrischen Impulse innerhalb einer Zeitspanne von 0,1 bis 100 ns, vorzugsweise innerhalb einer Zeitspanne von 1 ns bis 25 ns stattfindet; wobei die elektrischen Impulse eine Impulsdauer von 5 bis 5000 ns, vorzugsweise 50 ns bis 300 ns aufweisen; und wobei die elektrischen Impulse bei Erreichen der Zielspannung eine elektrische Feldstärke von 0,5 kV/cm bis 50 kV/cm, vorzugsweise 1,0 kV/cm bis 30 kV/cm aufweisen;
wobei die biologischen Zellen aus der Gruppe bestehend aus Bakterienzellen, Hefezellen, Pilzzellen, Mikroalgenzellen, Pflanzenzellen und Tierzellen ausgewählt werden, wobei die Tierzellen aus der Gruppe bestehend aus *Spodoptera frugiperda* und *Trichoplusia ni* ausgewählt werden.

4. Verfahren nach Anspruch 2 oder 3, wobei
- die Bakterienzellen aus der Gruppe bestehend aus *Escherichia coli* und *Corynebacterium glutamicum* ausgewählt werden;
- die Hefezellen aus der Gruppe bestehend aus *Pichia pastoris* und *Saccharomyces cerevisiae* ausgewählt werden;
- die Mikroalgenzellen aus der Gruppe bestehend aus *Galdieria sulphuraria* und *Aurantiochytrium limacinum* ausgewählt werden;
- die Pflanzenzellen aus der Gruppe bestehend aus *Hordeum vulgare* und *Oryza sativa* ausgewählt werden; und
- die Bakterienzellen aus der Gruppe bestehend aus *Spodoptera frugiperda* und *Trichoplusia ni* ausgewählt werden.

5. Verfahren nach einem der Ansprüche 1 bis 4, wobei Schritt (c) in einem an einen industriellen Maßstab angepassten Rezirkulationsprozess ausgeführt wird.

6. Verfahren nach einem der Ansprüche 1 bis 4, wobei Schritt (c) in einem Chargenprozess ausgeführt wird.

7. *In*-*vitro*-Verfahren zur Stimulation der Zellproliferation und/oder Erhöhung der Stoffwechselaktivität von biologischen Zellen, wobei das Verfahren die folgenden Schritte umfasst:
(a) Suspendieren von biologischen Zellen in einer elektrisch leitenden Flüssigkeit, um dadurch eine Suspension zu bilden,
(b) Positionieren der Suspension zwischen zwei Elektroden und
(c) Anlegen von 1 bis 100 elektrischen Impulsen, vorzugsweise 2 bis 30 elektrischen Impulsen zwischen den Elektroden, um dadurch die Zellproliferation zu stimulieren;
wobei eine Spannungserhöhung zwischen den beiden Elektroden von 10 % bis 90 % einer Zielspannung der elektrischen Impulse innerhalb einer Zeitspanne von 0,1 bis 100 ns, vorzugsweise innerhalb einer Zeitspanne von 1 ns bis 25 ns stattfindet; wobei die elektrischen Impulse eine Impulsdauer von 5 bis 5000 ns, vorzugsweise 50 ns bis 300 ns aufweisen; und wobei die elektrischen Impulse bei Erreichen der Zielspannung eine elektrische Feldstärke von 0,5 kV/cm bis 50 kV/cm, vorzugsweise 1,0 kV/cm bis 30 kV/cm aufweisen;
wobei Schritt (c) in einem an einen industriellen Maßstab angepassten Rezirkulationsprozess ausgeführt wird.

8. Verfahren nach Anspruch 7, wobei die biologischen Zellen aus der Gruppe bestehend aus Bakterienzellen, Hefezellen, Pilzzellen, Mikroalgenzellen, Pflanzenzellen, Tierzellen und Menschenzellen ausgewählt werden.

9. Verfahren nach Anspruch 8, wobei,
- die Bakterienzellen aus der Gruppe bestehend aus *Escherichia coli* und *Corynebacterium glutamicum* ausgewählt werden;
- die Hefezellen aus der Gruppe bestehend aus *Pichia pastoris* und *Saccharomyces cerevisiae* ausgewählt werden;
- die Mikroalgenzellen aus der Gruppe bestehend aus *Galdieria sulphuraria* und *Aurantiochytrium limacinum* ausgewählt werden;
- die Pflanzenzellen aus der Gruppe bestehend aus *Hordeum vulgare* und *Oryza sativa* ausgewählt werden; und
- die Bakterienzellen aus der Gruppe bestehend aus *Spodoptera frugiperda* und *Trichoplusia ni* ausgewählt werden.

## Revendications

1. Procédé *in vitro* d'augmentation d'activité métabolique de cellules biologiques, ledit procédé comprenant les étapes suivantes :
(a) la mise en suspension de cellules biologiques dans un liquide électroconducteur, formant ainsi une suspension,
(b) le positionnement de la suspension entre deux électrodes, et
(c) l'application de 1 à 100 impulsions électriques, de préférence 2 à 30 impulsions électriques, entre les électrodes, augmentant ainsi l'activité métabolique des cellules biologiques ;
dans lequel une augmentation de tension entre les deux électrodes allant de 10% à 90% d'une tension cible des impulsions électriques a lieu dans une période allant de 0,1 à 100 ns, de préférence dans une période allant de 1 ns à 25 ns ; où les impulsions électriques ont une durée d'impulsion comprise entre 5 et 5000 ns, de préférence entre 50 ns et 300 ns ; et où les impulsions électriques, lorsqu'elles atteignent la tension cible, ont une intensité de champ électrique allant de 0,5 kV/cm à 50 kV/cm, de préférence de 1,0 kV/cm à 30 kV/cm.

2. Procédé de la revendication 1, dans lequel les cellules biologiques sont choisies dans le groupe constitué par des cellules bactériennes, des cellules de levure, des cellules fongiques, des cellules de microalgues, des cellules végétales, des cellules animales et des cellules humaines.

3. Procédé *in vitro* de stimulation de prolifération cellulaire et/ou d'augmentation d'activité métabolique de cellules biologiques, ledit procédé comprenant les étapes suivantes :
(a) la mise en suspension de cellules biologiques dans un liquide électroconducteur, formant ainsi une suspension,
(b) le positionnement de la suspension entre deux électrodes, et
(c) l'application de 1 à 100 impulsions électriques, de préférence 2 à 30 impulsions électriques, entre les électrodes, stimulant ainsi la prolifération cellulaire ;
dans lequel une augmentation de tension entre les deux électrodes allant de 10% à 90% d'une tension cible des impulsions électriques a lieu dans une période allant de 0,1 à 100 ns, de préférence dans une période allant de 1 ns à 25 ns ; où les impulsions électriques ont une durée d'impulsion comprise entre 5 et 5000 ns, de préférence entre 50 ns et 300 ns ; et où les impulsions électriques, lorsqu'elles atteignent la tension cible, ont une intensité de champ électrique allant de 0,5 kV/cm à 50 kV/cm, de préférence de 1,0 kV/cm à 30 kV/cm ;
dans lequel les cellules biologiques sont choisies dans le groupe constitué par des cellules bactériennes, des cellules de levure, des cellules fongiques, des cellules de microalgues, des cellules végétales, et des cellules animales, dans lequel les cellules animales sont choisies dans le groupe constitué par *Spodoptera frugiperda* et *Trichoplusia ni.*

4. Procédé de la revendication 2 ou 3, dans lequel
- les cellules bactériennes sont choisies dans le groupe constitué par *Escherichia coli* et *Corynebacterium glutamicum ;*
- les cellules de levure sont choisies dans le groupe constitué par *Pichia pastoris* et *Saccharomyces cerevisiae ;*
- les cellules de microalgues sont choisies dans le groupe constitué par *Galdieria sulphuraria* et *Aurantiochytrium limacinum ;*
- les cellules végétales sont choisies dans le groupe constitué par *Hordeum vulgare* et *Oryza sativa* ; et
- les cellules animales sont choisies dans le groupe constitué par *Spodoptera frugiperda* et *Trichoplusia ni.*

5. Procédé de l'une quelconque des revendications 1 à 4, dans lequel l'étape (c) est réalisée dans un procédé de recirculation adapté à une échelle industrielle.

6. Procédé de l'une quelconque des revendications 1 à 4, dans lequel l'étape (c) est réalisée dans un procédé discontinu.

7. Procédé *in vitro* de stimulation de prolifération cellulaire et/ou d'augmentation d'activité métabolique de cellules biologiques, ledit procédé comprenant les étapes suivantes :
(a) la mise en suspension de cellules biologiques dans un liquide électroconducteur, formant ainsi une suspension,
(b) le positionnement de la suspension entre deux électrodes, et
(c) l'application de 1 à 100 impulsions électriques, de préférence 2 à 30 impulsions électriques, entre les électrodes, stimulant ainsi la prolifération cellulaire ;
dans lequel une augmentation de tension entre les deux électrodes allant de 10% à 90% d'une tension cible des impulsions électriques a lieu dans une période allant de 0,1 à 100 ns, de préférence dans une période allant de 1 ns à 25 ns ; où les impulsions électriques ont une durée d'impulsion comprise entre 5 et 5000 ns, de préférence entre 50 ns et 300 ns ; et où les impulsions électriques, lorsqu'elles atteignent la tension cible, ont une intensité de champ électrique allant de 0,5 kV/cm à 50 kV/cm, de préférence de 1,0 kV/cm à 30 kV/cm ;
dans lequel l'étape (c) est réalisée dans un procédé de recirculation adapté à une échelle industrielle.

8. Procédé de la revendication 7, dans lequel les cellules biologiques sont choisies dans le groupe constitué par des cellules bactériennes, des cellules de levure, des cellules fongiques, des cellules de microalgues, des cellules végétales, des cellules animales et des cellules humaines.

9. Procédé de la revendication 8, dans lequel
- les cellules bactériennes sont choisies dans le groupe constitué par *Escherichia coli* et *Corynebacterium glutamicum ;*
- les cellules de levure sont choisies dans le groupe constitué par *Pichia pastoris* et *Saccharomyces cerevisiae ;*
- les cellules de microalgues sont choisies dans le groupe constitué par *Galdieria sulphuraria* et *Aurantiochytrium limacinum ;*
- les cellules végétales sont choisies dans le groupe constitué par *Hordeum vulgare* et *Oryza sativa* ; et
- les cellules animales sont choisies dans le groupe constitué par *Spodoptera frugiperda* et *Trichoplusia ni.*
